(19) Europäisches Patentamt
European Patent Office
Office européen des brevets

(11) EP 4 527 329 A1

(12) **EUROPEAN PATENT APPLICATION**
published in accordance with Art. 153(4) EPC

(43) Date of publication:
26.03.2025 Bulletin 2025/13

(21) Application number: 23806995.9

(22) Date of filing: 17.05.2023

(51) International Patent Classification (IPC):
**A61B 18/14** *(2006.01)*

(52) Cooperative Patent Classification (CPC):
**A61B 18/12; A61B 18/14**

(86) International application number:
**PCT/CN2023/094825**

(87) International publication number:
**WO 2023/222044 (23.11.2023 Gazette 2023/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 17.05.2022 CN 202210541390

(71) Applicant: **Sichuan Jinjiang Electronic Medical
Device
Technology Co., Ltd.**
**Chengdu, Sichuan 610045 (CN)**

(72) Inventors:
• **DENG, Li**
**Chengdu, Sichuan 610045 (CN)**
• **HUANG, Yongjun**
**Chengdu, Sichuan 610045 (CN)**

(74) Representative: **Di Giovine, Paolo et al
Società Italiana Brevetti S.p.A.
Piazza di Pietra, 38-39
00186 Roma (IT)**

(54) **METHOD AND SYSTEM FOR IDENTIFYING CONTACT BETWEEN ELECTRODES AND TISSUE**

(57) The present invention discloses a method and system for identifying the contact between electrodes and a tissue. The method (700) comprises: acquiring an impedance between the electrodes on a body cavity invasion device at different frequencies (S710); determining, according to the acquired impedance between the electrodes, an impedance frequency response coefficient between the electrodes (S720); and determining, according to the impedance frequency response coefficient, the contact condition between the electrodes and a tissue in a body cavity (S730). The body cavity invasion device of the present invention comprises a catheter. A plurality of electrodes are disposed at a distal end of the invasive catheter. The contact condition includes whether the electrodes are in contact with the surface of the body cavity and the extent of the contact.

700

S710
Acquiring an impedance between the electrodes on a body cavity invasion device at different frequencies

S720
Determining, according to the acquired impedance between the electrodes, an impedance frequency response coefficient between the electrodes

S730
Determining, according to the impedance frequency response coefficient, a contact status between the electrode and the tissue in a body cavity

FIG. 7

EP 4 527 329 A1

## Description

### Cross-reference to Related Applications

The present application claims priority to Chinese Patent Application No. 202210541390.8, entitled "Method and System for Identifying Contact between Electrodes and Tissue", filed on May 17, 2022, which is incorporated herein by reference in its entirety.

### Technical Field

The present invention relates to electrophysiological ablation and circuitry, and particularly to a method and system for identifying contact between an electrode and tissue.

### Background

Currently, positioning of one or more invasion devices and determination of a relative position between the invasion device and to-be-diagnosed-and-treated tissue are usually involved in medical three-dimensional positioning systems. For example, in practice, it is necessary to position the invasion device within a body cavity, and in particular to identify contact between the invasion device and a surface of the body cavity. Generally, it is necessary to assess the contact between the invasion device within a heart chamber or a renal artery and the surface of the body cavity. More specifically, it is necessary to verify whether the invasion device is in contact with the tissue.

The three-dimensional positioning system with a contact indication function can reflect more realistically the relative position relationship between the invasion device and an intima surface of the body cavity. Contact indication application of the medical three-dimensional positioning system includes a contact indication of a catheter with a heart chamber wall and/or a blood vessel wall, and a contact indication of the catheter with a blood vessel inner wall of the renal artery.

Common invasion devices include, for example, a catheter, a sheath, and a puncture needle provided with one or more positioning electrodes. Based on the external shape, the catheter may include, for example, a basket-shaped catheter, a balloon-shaped catheter, a ring-shaped catheter, a petal-shaped catheter, a grid-shaped catheter, a line-shaped catheter, or the like.

Therefore, there is a need for a method and system for determining the relative position between the invasion device and the to-be-diagnosed-and-treated tissue and an extent of the contact therebetween, which is easy to operate in practice.

### Summary

The present invention aims to provide a system and method for positioning an invasion device in a body cavity, particularly relates to a method and system for identifying contact between an invasion device and a surface of a body cavity, and non-exclusively relates to a method and system for evaluating contact between an invasion device in a heart chamber or a renal artery and a surface of a body cavity. The invasion device involved in the present invention non-exclusively relates to invasion devices of a basket shape, a balloon shape, a ring shape, a petal shape, a grid shape and a line shape with similar principles.

According to a first aspect of the present invention, there is provided a method for identifying contact between an electrode and tissue. The method according to the first aspect of the present invention may include: acquiring an impedance between the electrodes on a body cavity invasion device at different frequencies; determining, according to the acquired impedance between the electrodes, an impedance frequency response coefficient between the electrodes; and determining, according to the impedance frequency response coefficient, a contact status between the electrode and the tissue in a body cavity.

Preferably, the electrode include a working electrode and a reference electrode. In the method according to the first aspect of the present invention, the acquiring an impedance between the electrodes at different frequencies may include: acquiring an impedance between the working electrodes, between the reference electrodes, and between the working electrode and the reference electrode at different frequencies.

Preferably, the working electrode may include an ablation electrode.

In the method according to the first aspect of the present invention, preferably, the determining a contact status between the electrode and the tissue in a body cavity may include: determining a contact status between the working electrode and the tissue in the body cavity.

Preferably, the body cavity invasion device may include a catheter, and the electrode is provided at a distal end of the catheter.

Preferably, the catheter may include at least one of a basket-shaped catheter, a balloon-shaped catheter, a ring-shaped catheter, a petal-shaped catheter, a grid-shaped catheter and a line-shaped catheter.

Preferably, the different frequencies may be selected according to a degree of difference how impedances of different tissues are in response to the frequencies.

Preferably, the different frequencies may range from 500 Hz to 100 kHz.

Preferably, the different frequencies may be two different frequencies.

In the method according to the first aspect of the present invention, the determining an impedance frequency response coefficient between the electrodes may include: calculating an impedance frequency response coefficient between the working electrodes and an impedance frequency response coefficient between the reference electrodes according to equations (1) and (2), respectively:

the impedance frequency response coefficient $\text{Coef}_{ij}$ between the working electrodes i and j is:

$$\text{Coef}_{ij} = a_{ij}\frac{\text{Impe}_{ij}^{Fr1}}{\text{Impe}_{ij}^{Fr2}} + b_{ij}\frac{\text{Real}_{ij}^{Fr1}}{\text{Real}_{ij}^{Fr2}} + c_{ij}\frac{\text{Imag}_{ij}^{Fr1}}{\text{Imag}_{ij}^{Fr2}} \quad \ldots\ldots\ldots\ldots\ldots\ldots \quad (1)$$

the impedance frequency response coefficient $\text{Coef}_{mn}$ between the reference electrodes m and n is:

$$\text{Coef}_{mn} = a_{mn}\frac{\text{Impe}_{mn}^{Fr1}}{\text{Impe}_{mn}^{Fr2}} + b_{mn}\frac{\text{Real}_{mn}^{Fr1}}{\text{Real}_{mn}^{Fr2}} + c_{mn}\frac{\text{Imag}_{mn}^{Fr1}}{\text{Imag}_{mn}^{Fr2}} \quad \ldots\ldots\ldots\ldots\ldots \quad (2)$$

the determining a contact status between the electrode and the tissue in a body cavity may include: calculating a contact index CI of the working electrodes i and j with the tissue according to equation (3):

$$CI = \frac{\left(\text{Coef}_{ij} - \text{Base}_{ij}\right)\big/\text{Dis}_{ij}}{\left(\text{Coef}_{mn} - \text{Base}_{mn}\right)\big/\text{Dis}_{mn}} \quad \ldots\ldots\ldots\ldots\ldots\ldots\ldots\ldots\ldots\ldots\ldots\ldots \quad (3)$$

In the above equations (1), (2) and (3), $\text{Impe}_{ij}^{Fr1}$ denotes an impedance measured between the working electrodes i and j at a first frequency Fr1, $\text{Impe}_{ij}^{Fr2}$ denotes an impedance measured between the working electrodes i and j at a second frequency Fr2, $\text{Real}_{ij}^{Fr1}$ denotes a real part of a complex impedance measured between the working electrodes i and j at the first frequency Fr1, $\text{Real}_{ij}^{Fr2}$ denotes a real part of a complex impedance measured between the working electrodes i and j at the second frequency Fr2, $\text{Imag}_{ij}^{Fr1}$ denotes an imaginary part of the complex impedance measured between the working electrodes i and j at the first frequency Fr1, $\text{Imag}_{ij}^{Fr2}$ denotes an imaginary part of the complex impedance measured between the working electrodes i and j at the second frequency Fr2, $\text{Impe}_{mn}^{Fr1}$ denotes an impedance measured between the reference electrodes m and n at the first frequency Fr1, $\text{Impe}_{mn}^{Fr2}$ denotes an impedance measured between the reference electrodes m and n at the second frequency Fr2, $\text{Real}_{mn}^{Fr1}$ denotes a real part of a complex impedance measured between the reference electrodes m and n at the first frequency Fr1, $\text{Real}_{mn}^{Fr2}$ denotes a real part of a complex impedance measured between the reference electrodes m and n at the second frequency Fr2, $\text{Imag}_{mn}^{Fr1}$ denotes an imaginary part of the complex impedance measured between the reference electrodes m and n at the first frequency Fr1, $\text{Imag}_{mn}^{Fr2}$ denotes an imaginary part of the complex impedance measured between the reference electrodes m and n at the second frequency Fr2, $a_{ij}$, $b_{ij}$, $c_{ij}$, $a_{mn}$, $b_{mn}$, $c_{mn}$ are weight coefficients, $Base_{ij}$ is a base of a frequency response between the working electrodes i and j, $Base_{mn}$ is a base of a frequency response between the reference electrodes m and n, $Dis_{ij}$ is a distance between the working electrodes i and j, and $Dis_{mn}$ is a distance between the reference electrodes m and n.

Preferably, the parameter $Base_{ij}$ and the parameter $Base_{mn}$ may have correlation with the parameter $Dis_{ij}$ and the parameter $Dis_{mn}$.

Preferably, the parameter $Base_{ij}$ and the parameter $Base_{mn}$ can be obtained by performing high-order regression modeling analysis with the parameter $Dis_{ij}$, the parameter $Dis_{mn}$, a parameter for electrode width and a parameter for electrode diameter.

Preferably, magnitudes of the weight coefficients $a_{ij}$, $b_{ij}$, $c_{ij}$, $a_{mn}$, $b_{mn}$, $c_{mn}$ may reflect weights of an impedance characteristic, a capacitive reactance characteristic and a comprehensive resistance-capacitance characteristic of the tissue or blood between the electrodes in an application environment, and $a_{ij} + b_{ij} + c_{ij} = 1$, $a_{mn} + b_{mn} + C_{mn} = 1$.

In the method according to the first aspect of the present invention, preferably, the method may further include: displaying the contact status between the electrode and the tissue in the body cavity with a color difference.

According to a second aspect of the present invention, there is provided a system for identifying contact between an electrode and tissue. The system may include: an acquiring unit configured to acquire an impedance between the electrodes on a body cavity invasion device at different frequencies; an impedance frequency response coefficient determining unit configured to determine, according to the acquired impedance between the electrodes, an impedance frequency response coefficient between the electrodes; and a contact status determining unit configured to determine, according to the impedance frequency response coefficient, a contact status between the electrode and the tissue in a body cavity.

Preferably, the electrode may include a working electrode and a reference electrode. In the system according to the second aspect of the present invention, the acquiring unit may be configured to: acquire an impedance between the working electrodes, between the reference electrodes, and between the working electrode and the reference electrode at different frequencies.

Preferably, the working electrode may include an ablation electrode.

In the system according to the second aspect of the present invention, preferably, the contact status determining unit may be configured to: determine a contact status between the working electrode and the tissue in the body cavity.

Preferably, the body cavity invasion device may include a catheter, and the electrode is provided at a distal end of the catheter.

Preferably, the catheter may include at least one of a basket-shaped catheter, a balloon-shaped catheter, a ring-shaped catheter, a petal-shaped catheter, a grid-shaped catheter, and a line-shaped catheter.

Preferably, the different frequencies may be selected according to a degree of difference how impedances of different tissues are in response to the frequencies.

Preferably, the different frequencies may range from 500 Hz to 100 kHz.

Preferably, the different frequencies may be two different frequencies.

In the system according to the second aspect of the present invention, the impedance frequency response coefficient determining unit may be configured to: calculate an impedance frequency response coefficient between the working electrodes and an impedance frequency response coefficient between the reference electrodes according to equations (1) and (2), respectively:

the impedance frequency response coefficient $Coef_{ij}$ between the working electrodes i and j is:

$$\text{Coef}_{ij} = a_{ij} \frac{\text{Impe}_{ij}^{Fr1}}{\text{Impe}_{ij}^{Fr2}} + b_{ij} \frac{\text{Real}_{ij}^{Fr1}}{\text{Real}_{ij}^{Fr2}} + c_{ij} \frac{\text{Imag}_{ij}^{Fr1}}{\text{Imag}_{ij}^{Fr2}} \quad \ldots\ldots\ldots\ldots\ldots\ldots \quad （1）$$

the impedance frequency response coefficient $Coef_{mn}$ between the reference electrodes m and n is:

$$\text{Coef}_{mn} = a_{mn} \frac{\text{Impe}_{mn}^{Fr1}}{\text{Impe}_{mn}^{Fr2}} + b_{mn} \frac{\text{Real}_{mn}^{Fr1}}{\text{Real}_{mn}^{Fr2}} + c_{mn} \frac{\text{Imag}_{mn}^{Fr1}}{\text{Imag}_{mn}^{Fr2}} \quad \ldots\ldots\ldots\ldots\ldots \quad （2）$$

the contact status determining unit may be configured to: calculate a contact index CI of the working electrodes i and j with the tissue according to equation (3):

$$CI = \frac{\left(Coef_{ij} - Base_{ij}\right)\big/ Dis_{ij}}{\left(Coef_{mn} - Base_{mn}\right)\big/ Dis_{mn}} \quad ............................ \quad (3)$$

In the above equations (1), (2) and (3), $Impe_{ij}^{Fr1}$ denotes an impedance measured between the working electrodes i and j at a first frequency Fr1, $Impe_{ij}^{Fr2}$ denotes an impedance measured between the working electrodes i and j at a second frequency Fr2, $Real_{ij}^{Fr1}$ denotes a real part of a complex impedance measured between the working electrodes i and j at the first frequency Fr1, $Real_{ij}^{Fr2}$ denotes a real part of a complex impedance measured between the working electrodes i and j at the second frequency Fr2, $Imag_{ij}^{Fr1}$ denotes an imaginary part of the complex impedance measured between the working electrodes i and j at the first frequency Fr1, $Imag_{ij}^{Fr2}$ denotes an imaginary part of the complex impedance measured between the working electrodes i and j at the second frequency Fr2, $Impe_{mn}^{Fr1}$ denotes an impedance measured between the reference electrodes m and n at the first frequency Fr1, $Impe_{mn}^{Fr2}$ denotes an impedance measured between the reference electrodes m and n at the second frequency Fr2, $Real_{mn}^{Fr1}$ denotes a real part of a complex impedance measured between the reference electrodes m and n at the first frequency Fr1, $Real_{mn}^{Fr2}$ denotes a real part of a complex impedance measured between the reference electrodes m and n at the second frequency Fr2, $Imag_{mn}^{Fr1}$ denotes an imaginary part of the complex impedance measured between the reference electrodes m and n at the first frequency Fr1, $Imag_{mn}^{Fr2}$ denotes an imaginary part of the complex impedance measured between the reference electrodes m and n at the second frequency Fr2, $a_{ij}$, $b_{ij}$, $c_{ij}$, $a_{mn}$, $b_{mn}$, $c_{mn}$ are weight coefficients, $Base_{ij}$ is a base of a frequency response between the working electrodes i and j, $Base_{mn}$ is a base of a frequency response between the reference electrodes m and n, $Dis_{ij}$ is a distance between the working electrodes i and j, and $Dis_{mn}$ is a distance between the reference electrodes m and n.

Preferably, the parameter $Base_{ij}$ and the parameter $Base_{mn}$ may have correlation with the parameter $Dis_{ij}$ and the parameter $Dis_{mn}$.

Preferably, the parameter $Base_{ij}$ and the parameter $Base_{mn}$ can be obtained by performing high-order regression modeling analysis with the parameter $Dis_{ij}$, the parameter $Dis_{mn}$, a parameter for electrode width and a parameter for electrode diameter.

Preferably, magnitudes of the weight coefficients $a_{ij}$, $b_{ij}$, $c_{ij}$, $a_{mn}$, $b_{mn}$, $c_{mn}$ may reflect weights of an impedance characteristic, a capacitive reactance characteristic and a comprehensive resistance-capacitance characteristic of the tissue or blood between the electrodes in an application environment, and $a_{ij} + b_{ij} + c_{ij} = 1$, $a_{mn} + b_{mn} + C_{mn} = 1$.

In the system according to the second aspect of the present invention, preferably, the system may further include: a display unit configured to display the contact status between the electrode and the tissue in the body cavity with a color difference.

According to a third aspect of the present invention, there is provided a computer readable medium having stored thereon instructions executable by a processor, the instructions, when executed by the processor, causing the processor to perform the method for identifying contact between an electrode and tissue according to the first aspect of the present invention.

The present invention discloses the method and system for identifying contact between an invasion device and a surface of a body cavity in the field of positioning of the invasion device in the body cavity. The system includes an invasive catheter with a plurality of electrodes provided at a distal end thereof. The system acquires impedance information of the electrode relative to a reference at the plurality of frequencies, obtains the frequency response coefficient of the electrode based on the impedance information at the plurality of frequencies, and then obtains the contact index (or referred to as a sticking index) CI based on an indication of the frequency response coefficient. The CI indicates whether the electrode is in contact with the surface of the body cavity and an extent of the contact.

## Brief Description of the Drawings

The present disclosure includes the accompanying drawings, which are incorporated in and constitute a part of the specification, and together with the specification, illustrate various exemplary embodiments, features, and aspects of the present disclosure, and serve to explain the principles of the present disclosure. The present invention will be more fully understood from the following detailed description in combination with the accompanying drawings in which like elements are numbered in a similar manner. In the drawings:

FIG. 1 shows relative dielectric constants of blood, blood vessels and a heart at different frequencies.

FIG. 2 shows conductivities of blood, blood vessels and a heart at different frequencies.

FIG. 3A is a schematic diagram of an annular invasive catheter.

FIG. 3B is a schematic diagram showing application of the annular invasive catheter.

FIG. 4A is a schematic diagram of a petaloid invasive catheter.

FIG. 4B is a schematic diagram showing application of the petaloid invasive catheter.

FIG. 5 is a flow chart of a method for identifying contact between an electrode and tissue according to a preferred embodiment of the present invention.

FIG. 6 is a schematic diagram showing displaying of a contact identification result.

FIG. 7 is a flow chart of a method for identifying contact between an electrode and tissue according to an embodiment of the present invention.

FIG. 8 is a schematic block diagram of a system for identifying contact between an electrode and tissue according to an embodiment of the present invention.

## Detailed Description

The technical solution of the present invention will be described in further detail below with reference to the embodiments and drawings, but the present invention is not limited to the following embodiments.

Various exemplary embodiments, features and aspects of the present disclosure will be described in detail below with reference to the accompanying drawings. In the drawings, like reference numbers indicate functionally identical or similar elements. While various aspects of the embodiments are shown in the drawings, the drawings are not necessarily drawn to scale unless specifically indicated.

The word "exemplary" used exclusively herein means "serving as an example, embodiment, or illustration". Any embodiments described herein as "exemplary" is not necessarily to be construed as preferable or advantageous over other embodiments.

In addition, in order to better illustrate the present disclosure, numerous specific details are given in the following specific embodiments. It should be understood by those skilled in the art that the present disclosure may be practiced without some of these specific details. In some instances, methods, means, elements and circuits that are well known to those skilled in the art are not described in detail, so as to highlight the subject matter of the present disclosure.

In general, the present invention provides a method and system for identifying contact between electrodes and tissue. The implementation of the method and system according to the present invention is based on the principle that impedances of different biological tissues in response to frequencies vary.

As the impedance is involved, it is necessary to acquire an impedance, or referred as "measure an impedance". Hereinafter, the terms "acquire" and "measure" may be used interchangeably. Acquisition of an impedance or measurement of an impedance represents obtaining of a numerical value of the impedance.

The manner of acquiring the impedance may include, but is not limited to, acquisition of an impedance between the electrodes, an impedance between the electrode and the reference, or an impedance between the references at different frequencies. Here, the electrode refers to an electrode on an invasion device, such as an ablation electrode, a mapping electrode, or the like, and more specifically, refers to a working electrode which performs an actual treatment or detection function. The reference may be a ground level, or a reference level outside a body cavity. The reference may also be a reference electrode. Hereinafter, in order to distinguish the electrode from the reference, the electrode may be classified into two types: a working electrode and a reference electrode. In the case of treatment using the invasion device, the working electrode on the invasion device may also be referred to as a treatment electrode. Thus, the said acquisition of the impedance at different frequencies can be explained in more detail as acquisition of the impedance between the working electrodes, between the reference electrodes, and between the working electrode and the reference electrode at different frequencies.

As described above, in a case where the invasion device is an ablation catheter, the working electrode may be an ablation electrode.

In embodiments where the body cavity invasion device is a catheter, both the working electrode and the reference electrode may be provided at a distal end of the catheter. In some embodiments, the reference electrode may be provided at other positions, for example, outside the body.

The catheter may be any one or at least one of a basket-shaped catheter, a balloon-shaped catheter, a ring-shaped catheter, a petal-shaped catheter, a grid-shaped catheter, and a line-shaped catheter.

According to the present invention, the acquired impedance information is used to solve a frequency response coefficient of the electrode. In a preferred embodiment, the frequency response coefficient of the electrode include a frequency response coefficient between the working electrodes and a frequency response coefficient between the reference electrodes.

Based on the frequency response coefficient of the electrode, a contact status between the electrode and the tissue in the body cavity may be determined. For example, it is possible to determine whether the electrode is in contact with the tissue wall of the body cavity and the extent of the contact. The involved electrode for determining whether it is in contact with the tissue in the body cavity is the working electrode.

The acquisition of the impedance at different frequencies includes time-division acquisition and frequency-division acquisition.

The procedure of the so-called time-division acquisition may be the following: in the case of the acquisition of the impedance information at several (for example, n) frequencies, the acquisition of the impedance information at the 1st frequency, at the 2nd frequency, ... , is performed in sequence, until the acquisition of impedance information at the nth frequency. After this round of the acquisition is finished, or at regular intervals, the acquisition starts from the 1st frequency again, that is, the acquisition of the impedance information at the 1st frequency, the 2nd frequency, ... , is performed in sequence, until the acquisition of the impedance information at the nth frequency. In this way, the time-division acquisition is performed successively and repeatedly.

The following description of the present application is given with the frequency-division acquisition as an example.

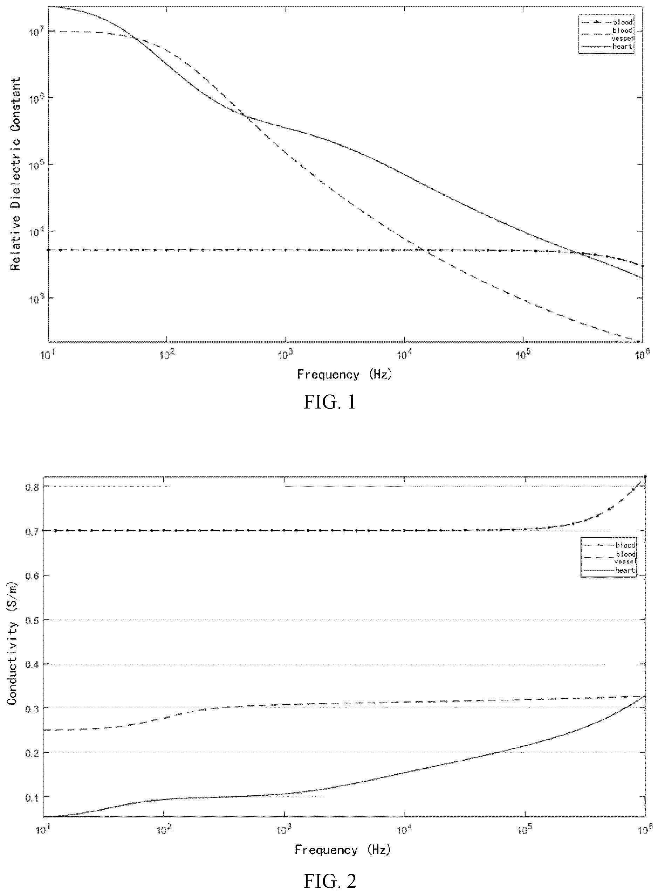

The acquisition frequency may be selected based on a degree of difference how impedances of two or more tissues are in response to the frequencies. FIG. 1 shows relative dielectric constants of blood, blood vessels and a heart at different frequencies. FIG. 2 shows conductivities of the blood, the blood vessels and the heart at different frequencies. The plural impedance acquisition frequencies can be selected from a range of 500 Hz to 100 kHz, for example.

The acquisition frequency can be determined according to an actual application scenario. For example, the scenario shown in FIGs. 1 and 2 is identification of contact between the electrode and tissue in a heart chamber and a blood vessel, and depending on different impedances in response to frequencies for the cardiac muscle, the blood vessel wall and the blood, the appropriate acquisition frequency is selected in accordance with the principle that the impedance difference among the three at the selected frequency shall be as large as possible. Here, the large difference can be understood as high resolution of tissue distinction.

It shall be appreciated by those skilled in the art that FIGs. 1 and 2 reflect the principle that the impedance in response to frequencies vary among different tissue, and the two figures are intended to depict the same principle but in different ways: FIG. 1 is depicted from the perspective of a relative dielectric constant, and FIG. 2 is depicted from the perspective of a conductivity.

Invasion Device

The invasion device of the present invention is discussed below.

The invasion device, i.e., the body cavity invasion device, refers to a device which can invade into the body cavity.

In a preferred embodiment of the present invention, the invasion device is an invasive catheter. The electrode is provided at the distal end of the invasive catheter. An object of the present invention is to determine whether the electrode (for example, an ablation electrode) on the invasive catheter is in contact with the tissue in the body cavity (for example, a wall or surface of the body cavity) and the extent of the contact.

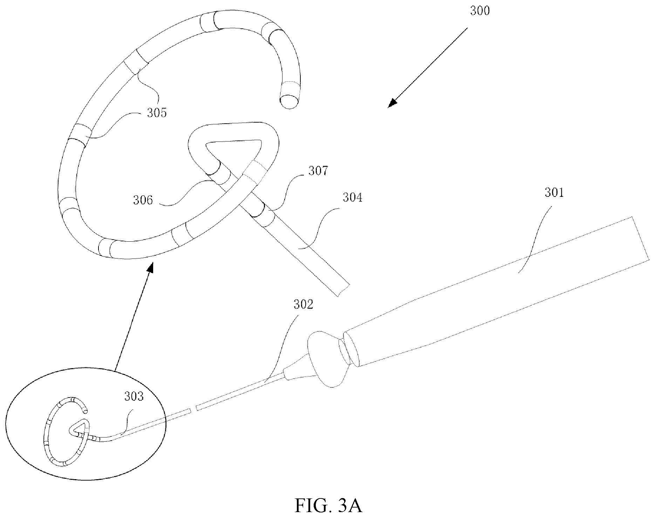

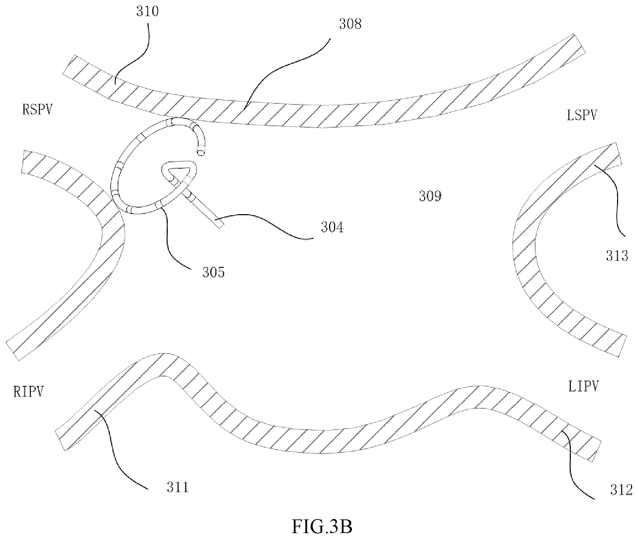

FIG. 3A is a schematic diagram of an annular invasive catheter. FIG. 3B is a schematic diagram showing application of the annular invasive catheter.

When the invasive catheter is the annular invasive catheter as shown in FIG. 3A, the catheter 300 includes an operating handle 301, a catheter proximal end 302, a catheter distal end 303, a catheter body 304, a torus electrode (working electrode) 305 and reference electrodes 306, 307.

As shown in FIG. 3B, in the environment where the annular catheter invades into the body cavity, it may be in contact with or in proximity to the following tissue or organs: the left atrial wall 308, the left atrial chamber 309, the right superior pulmonary vein 310, the right inferior pulmonary vein 311, the left inferior pulmonary vein 312, and the left superior pulmonary vein 313.

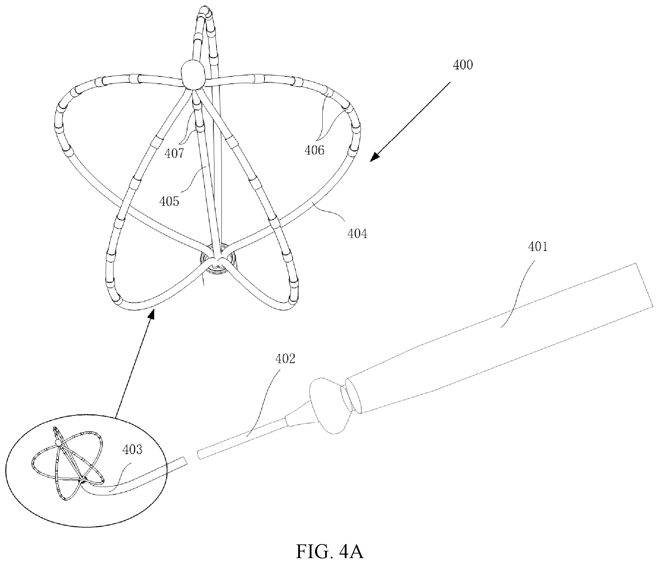

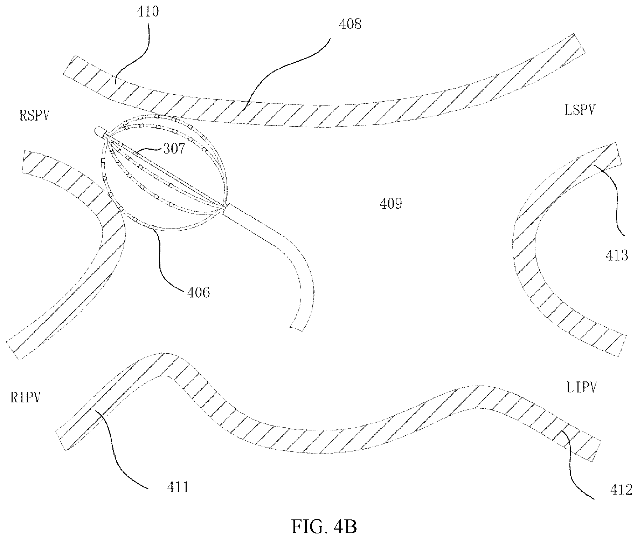

FIG. 4A is a schematic diagram of a petaloid invasive catheter. FIG. 4B is a schematic diagram showing application of the petaloid invasive catheter.

When the invasive catheter is the petaloid invasive catheter as shown in FIG. 4A, the catheter 400 includes: an operating handle 401, a catheter proximal end 402, a catheter distal end 403, a petal edge 404, a retractable pull rod 405, a petal electrode (working electrode) 406 and a pull rod reference electrode 407.

As shown in FIG. 4B, in the environment where the petaloid catheter invades into the body cavity, it may be in contact with or in proximity to the following tissue or organs: the left atrial wall 408, the left atrial chamber 409, the right superior pulmonary vein 410, the right inferior pulmonary vein 411, the left inferior pulmonary vein 412, and the left superior pulmonary vein 413.

The design of the invasive catheter ensures that the reference electrode is not in contact with the tissue under most conditions during an operation, making it reasonable as a reference for determining whether other electrodes (working electrodes) are sticking against. Compared with a method for detecting contact between the electrode and tissue based on impedance or phase data statistical analysis, it has a higher reliability.

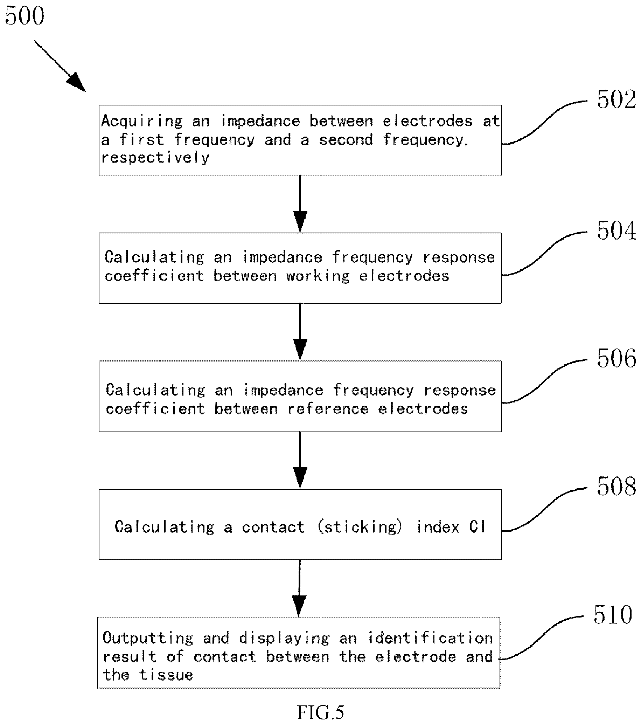

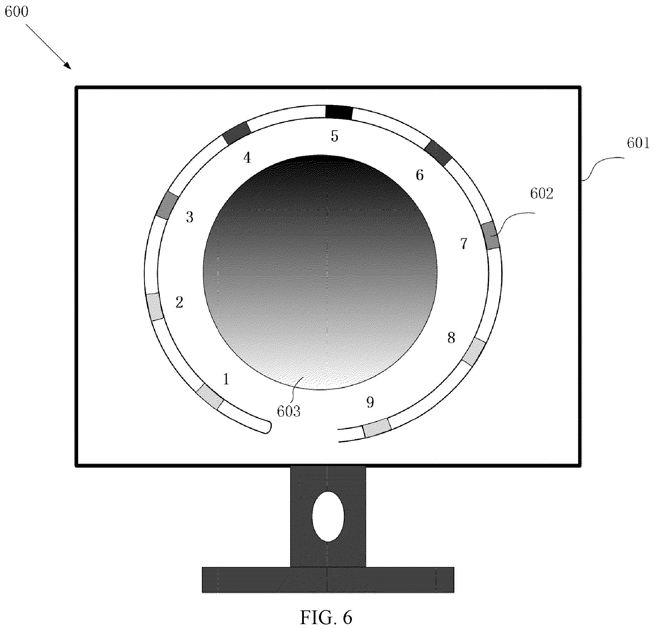

FIGs. 5 and 6 show a determination process and a display result of a contact status between the working electrode on the invasion device and the tissue more specifically. A more detailed explanation is given below.

Method for Identifying Contact

The method for identifying contact between an electrode and tissue according to an embodiment of the present invention is described below from a more general perspective.

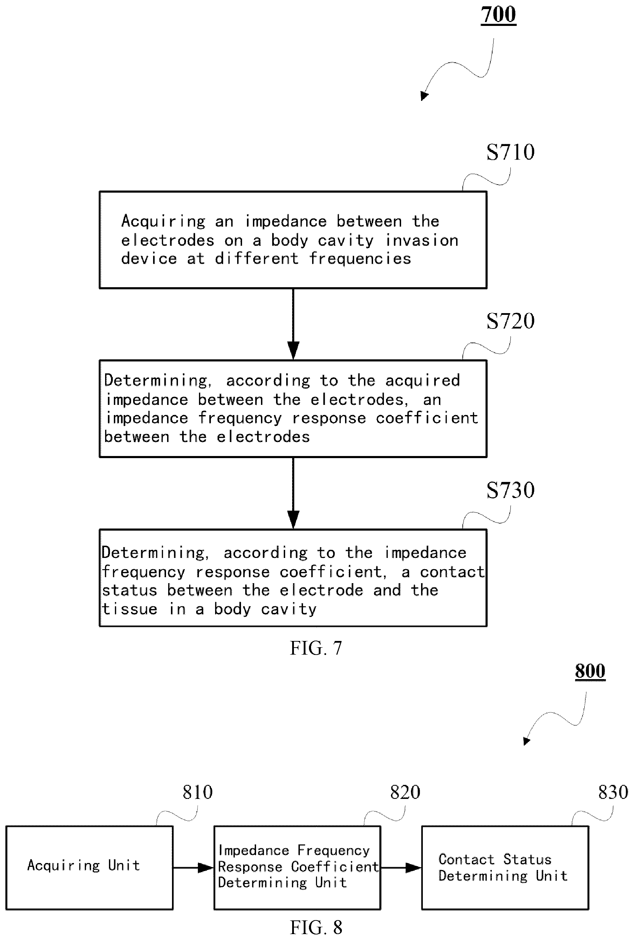

FIG. 7 is a flow chart of the method for identifying contact between an electrode and tissue according to the embodiment of the present invention.

As shown in FIG. 7, the method 700 for identifying contact between the electrode and tissue begins at step S710, where an impedance between the electrodes on a body cavity invasion device are acquired at different frequencies.

At step S710, the different acquisition frequencies are selected according to a degree of difference how impedances of different tissues are in response to the frequencies. Generally, the different acquisition frequencies range from 500 Hz to 100 kHz. In a preferred embodiment, the "different frequencies" refers to two different frequencies.

The acquisition of impedance at two frequencies is described as an example. The acquisition is performed at a first frequency to obtain a first impedance and is performed at a second frequency to obtain a second impedance. In a practical application process, it is possible to acquire impedance information at more frequencies. A common impedance acquisition process includes a time-division acquisition and a frequency-division acquisition. The flow of the time-division acquisition has been described above, and the frequency-division acquisition is adopted in the exemplary embodiment of the present application.

In a preferred embodiment of the present invention, the impedance acquisition is performed for the annular catheter shown in FIG. 3A. In the method according to the present invention, at step S710, the impedances between the adjacent torus electrodes 305 are acquired at the first frequency and the second frequency, respectively; the impedances between the reference electrodes 306, 307 are acquired at the first frequency and the second frequency, respectively. In particular, the relationship of the following distances has been taken into account during the design of the catheter: the distance between the adjacent electrodes 305 and the distance between the reference electrodes 306, 307; for example, these two distances meet an equal relationship or a proportional relationship, and the relationship can be used as a parameter for subsequently identifying contact between the electrode and the tissue. The reference electrode is not limited to the two reference electrodes described herein, and more than two reference electrodes may be arranged, so as to establish a linear or non-linear relationship for different reference electrode distances and the impedances.

In another preferred embodiment of the present invention, the impedance acquisition is performed for the petaloid catheter shown in FIG. 4A. In the method according to the present invention, at step S710, the impedances between the adjacent electrodes 406 on the torus pedal edge 404 are acquired at the first frequency and the second frequency, respectively; the impedances between the reference electrodes 407 are acquired at the first frequency and the second frequency, respectively. In particular, the relationship of the following distances has been taken into account during the design of the catheter: the distance between the adjacent electrodes 406 and the distance between the reference electrodes 407; for example, these two distances meet an equal relationship or a proportional relationship, and the relationship can be used as a parameter for subsequently identifying contact between the electrode and the tissue. The reference electrode is not limited to the two reference electrodes described herein, and more than two reference electrodes may be arranged on the retractable pull rod 405, so as to establish a linear or non-linear relationship for different reference electrode distances and the impedances.

Returning to FIG. 7, next, at step S720, an impedance frequency response coefficient between the electrodes is determined according to the impedance between the electrodes acquired at step S710.

Then, at step S730 of FIG. 7, a contact status between the electrode and the tissue in the body cavity is determined according to the impedance frequency response coefficient.

At step S730, in a preferred embodiment, it is the contact status between the working electrode (for example, the adjacent torus electrodes 305 shown in FIG. 3A, or the adjacent electrodes 406 on the torus petal edge 404 shown in FIG. 4A) and the tissue in the body cavity that is required to be determined, instead of the contact status between the reference electrode (for example, the reference electrodes 306, 307 shown in FIG. 3A, or the pull rod reference electrodes 407 shown in FIG. 4A) and the tissue in the body cavity.

Operations of step S720 and step S730 of FIG. 7 in a preferred embodiment of the present invention are explained below by referring to FIG. 5.

Taking the identification of the contact between the electrode and the tissue by acquiring impedances at two frequencies as an example, a specific process is shown in FIG. 5.

FIG. 5 is a flow chart of the method for identifying contact between an electrode and tissue according to a preferred embodiment of the present invention.

As shown in FIG. 5, the method 500 for identifying contact between the electrode and tissue begins at step 502, where the impedance between the electrodes is acquired at the first frequency and the second frequency, respectively, as described above.

Then, at step 504, an impedance frequency response coefficient between working electrodes is calculated. At step 506, an impedance frequency response coefficient between reference electrodes is calculated. Next, at step 508, a contact index of the electrode with the tissue is calculated. Finally, at step 510, whether the invasive catheter electrode is in contact with the tissue and the extent of the contact are output and displayed.

More specifically, the required contact status between the working electrode and the tissue can be finally obtained by the following calculation formulas.

(1) The impedance frequency response coefficient between the working electrodes i and j:

$$\text{Coef}_{ij} = a_{ij}\frac{\text{Impe}_{ij}^{Fr1}}{\text{Impe}_{ij}^{Fr2}} + b_{ij}\frac{\text{Real}_{ij}^{Fr1}}{\text{Real}_{ij}^{Fr2}} + c_{ij}\frac{\text{Imag}_{ij}^{Fr1}}{\text{Imag}_{ij}^{Fr2}} \quad \dots\dots\dots\dots\dots\dots \quad (1)$$

(2) The impedance frequency response coefficient between the reference electrodes m and n:

$$\text{Coef}_{mn} = a_{mn}\frac{\text{Impe}_{mn}^{Fr1}}{\text{Impe}_{mn}^{Fr2}} + b_{mn}\frac{\text{Real}_{mn}^{Fr1}}{\text{Real}_{mn}^{Fr2}} + c_{mn}\frac{\text{Imag}_{mn}^{Fr1}}{\text{Imag}_{mn}^{Fr2}} \quad \dots\dots\dots\dots\dots \quad (2)$$

(3) The contact index CI of the working electrodes i and j with the tissue:

$$\text{CI} = \frac{\left(\text{Coef}_{ij} - \text{Base}_{ij}\right)\big/\text{Dis}_{ij}}{\left(\text{Coef}_{mn} - \text{Base}_{mn}\right)\big/\text{Dis}_{mn}} \quad \dots\dots\dots\dots\dots\dots\dots\dots\dots\dots\dots\dots\dots \quad (3)$$

In the above equations (1), (2) and (3), $\text{Impe}_{ij}^{Fr1}$ denotes an impedance measured between the working electrodes i and j at a first frequency Fr1, $\text{Impe}_{ij}^{Fr2}$ denotes an impedance measured between the working electrodes i and j at a second frequency Fr2, $\text{Real}_{ij}^{Fr1}$ denotes a real part of a complex impedance measured between the working electrodes i and j at the first frequency Fr1, $\text{Real}_{ij}^{Fr2}$ denotes a real part of a complex impedance measured between the working electrodes i and j at the second frequency Fr2, $\text{Imag}_{ij}^{Fr1}$ denotes an imaginary part of the complex impedance measured between the working electrodes i and j at the first frequency Fr1, $\text{Imag}_{ij}^{\text{Fr2}}$ denotes an imaginary part of the complex impedance measured between the working electrodes i and j at the second frequency Fr2, $\text{Impe}_{mn}^{\text{Fr1}}$ denotes an impedance measured between the reference electrodes m and n at the first frequency Fr1, $\text{Impe}_{mn}^{\text{Fr2}}$ denotes an impedance measured between the reference electrodes m and n at the second frequency Fr2, $\text{Real}_{mn}^{\text{Fr1}}$ denotes a real part of a complex impedance measured between the reference electrodes m and n at the first frequency Fr1, $\text{Real}_{mn}^{\text{Fr2}}$ denotes a real part of a complex impedance measured between the reference electrodes m and n at the second frequency Fr2, $\text{Imag}_{mn}^{\text{Fr1}}$ denotes an imaginary part of the complex impedance measured between the reference electrodes m and n at the first frequency Fr1, $\text{Imag}_{mn}^{\text{Fr2}}$ denotes an imaginary part of the complex impedance measured between the reference electrodes m and n at the second frequency Fr2, $a_{ij}$, $b_{ij}$, $c_{ij}$, $a_{mn}$, $b_{mn}$, $c_{mn}$ are weight coefficients, $\text{Base}_{ij}$ is a base of a frequency response between the working electrodes i and j, $\text{Base}_{mn}$ is a base of a frequency response between the reference electrodes m and n, $\text{Dis}_{ij}$ is a distance between the working electrodes i and j, and $\text{Dis}_{mn}$ is a distance between the reference electrodes m and n.

The parameters Base and Dis have correlation. That is, the parameter $\text{Base}_{ij}$ and the parameter $\text{Base}_{mn}$ have correlation with the parameter $\text{Dis}_{ij}$ and the parameter $\text{Dis}_{mn}$, respectively.

Furthermore, electrode parameters, such as a width W and a diameter D, are also influence factors for the parameters Base. The parameters Base are specific parameters of the catheter, and are input to the system as a known value. The specific parameters Base may be obtained by performing high-order regression modeling analysis with the parameters Dis, W and D. That is, the parameter $\text{Base}_{ij}$ and the parameter $\text{Base}_{mn}$ are obtained by performing high-order regression modeling analysis with the parameter $\text{Dis}_{ij}$, the parameter $\text{Dis}_{mn}$, the parameter for electrode width and the parameter for electrode diameter.

Magnitudes of the weight coefficients $a_{ij}$, $b_{ij}$, $c_{ij}$, $a_{mn}$, $b_{mn}$, $c_{mn}$ reflect weights of an impedance characteristic, a capacitive reactance characteristic and a comprehensive resistance-capacitance characteristic of the tissue or blood between the electrodes in an application environment, and $a_{ij} + b_{ij} + c_{ij} = 1$, $a_{mn} + b_{mn} + C_{mn} = 1$.

At step 510, based on the contact index (or called a sticking index) CI, it is determined whether the electrode is in contact with the tissue by comparison with a preset threshold limit; and it may also be determined the extent of the contact between the electrode and the tissue by comparison with a plurality of preset threshold limits.

Furthermore, it is possible to display the contact status between the electrode and the tissue in the body cavity by a color difference.

FIG. 6 is a schematic diagram showing the displaying of the contact identification result.

As shown in FIG. 6, a screen 601 of a display 600 reflects an indication of whether the electrode is in contact with the tissue, and the color of the electrode 602 indicates the extent of the contact between the electrode and the tissue; a central part 603 shows the current sticking trend and which electrodes are sticking to the tissue. Different striking colors are used to annotate the sticking and non-sticking states, and meanwhile, indicate the magnitude of the sticking index value indirectly, so as to reflect the extent of the contact between the electrode and the tissue.

Contact Identification System

FIG. 8 is a schematic block diagram of the system for identifying contact between an electrode and tissue according to the embodiment of the present invention.

As shown in FIG. 8, the system 800 for identifying contact between the electrode and tissue includes an acquiring unit 810, an impedance frequency response coefficient determining unit 820 and a contact status determining unit 830.

The acquiring unit 810 is configured to acquire an impedance between the electrodes on a body cavity invasion device at different frequencies.

The body cavity invasion device includes a catheter, and the electrode is provided at a distal end of the catheter.

The catheter may be at least one or any one of a basket-shaped catheter, a balloon-shaped catheter, a ring-shaped catheter, a petal-shaped catheter, a grid-shaped catheter, and a line-shaped catheter.

The electrode described herein include a working electrode (or treatment electrode in a treatment scenario) and a reference electrode. For example, the working electrode may be an ablation electrode. Thus, the acquiring unit 810 may be configured to: acquire an impedance between the working electrodes, between the reference electrodes, and between the working electrode and the reference electrode at different frequencies.

The different frequencies may be selected according to a degree of difference how impedances of different tissues are in response to the frequencies.

The different frequencies range from 500 Hz to 100 kHz.

In a preferred embodiment, the different frequencies are two different frequencies.

The impedance frequency response coefficient determining unit 820 is configured to determine, according to the impedance between the electrodes acquired by the acquiring unit 810, an impedance frequency response coefficient between the electrodes.

The impedance frequency response coefficient determining unit 820 may calculate an impedance frequency response coefficient between the working electrodes and an impedance frequency response coefficient between the reference electrodes according to the above equations (1) and (2), respectively.

The contact status determining unit 830 is configured to determine, according to the impedance frequency response coefficient, a contact status between the electrode and the tissue in a body cavity. Specifically, the contact status determining unit may be configured to determine a contact status between the working electrode and the tissue in the body cavity.

More specifically, the contact status determining unit 830 may calculate a contact index CI of the working electrodes i and j with the tissue according to the above equation (3).

The system 800 may further include a display unit (not shown) configured to display the contact status between the electrode and the tissue in the body cavity by a color difference.

It will be appreciated by those skilled in the art that the technical means, steps and units described above may be arbitrarily combined to achieve the purpose of the present invention unless it is impossible to combine them logically or physically.

Computer Program and Computer Readable Medium

Furthermore, it will be appreciated by those skilled in the art that the method according to the present disclosure may be implemented as a computer program. As described above with reference to the drawings, the method according to the above-mentioned embodiments is performed by one or more programs, including instructions to cause a computer or processor to perform an algorithm described with reference to the drawings. These programs may be stored using various types of non-transitory computer readable media and provided to the computer or processor. The non-transitory computer readable media include various types of tangible storage media. Examples of the non-transitory computer readable medium include a magnetic recording medium (such as a floppy disk, a magnetic tape, and a hard disk drive), a magneto-optical recording medium (such as a magneto-optical disk), a Compact Disc-Read-Only Memory (CD-ROM), a CD-R, a CD-R/W, and a semiconductor memory (such as a ROM, a programmable ROM (PROM),an erasable PROM (EPROM), a flash ROM, and a Random Access Memory (RAM)). Further, these programs may be provided to the computer by using various types of transitory computer readable media. Examples of the transitory computer readable medium include an electric signal, an optical signal, and an electromagnetic wave. The transitory computer readable medium may be configured to provide the program to the computer through a wired communication path, such as an electric wire and an optical fiber, or a wireless communication path.

For example, an embodiment of the present disclosure may provide a device for identifying contact between an electrodes and tissue. The device includes a processor and a memory. The memory has stored therein a computer program which, when executed by the processor, implements the method for identifying contact between an electrode and tissue described above.

Therefore, according to the present disclosure, it is possible to further propose a computer program or a computer readable medium having stored thereon instructions executable by a processor, the instructions, when executed by the processor, causing the processor to perform the method for identifying contact between electrodes and tissue described before.

While various embodiments of the present disclosure have been described above, the above description is intended to be illustrative, and not exhaustive, and the scope of the present invention is not limited to the embodiments described above. Many modifications and variations will be apparent to those skilled in the art without departing from the spirit and scope of the present invention. That is, various changes and modifications in form and in detail may be made to the present invention by those skilled in the art, all of which are considered to fall into the protection scope of the present invention. The terminology used herein was chosen to best explain the principles of the embodiments, the practical application or technical improvement over technologies found in the marketplace, or to enable those skilled in the art to understand the embodiments disclosed herein.

**Claims**

**1.** A method for identifying contact between an electrode and tissue, comprising:

acquiring an impedance between the electrodes on a body cavity invasion device at different frequencies;
determining, according to the acquired impedance between the electrodes, an impedance frequency response coefficient between the electrodes; and
determining, according to the impedance frequency response coefficient, a contact status between the electrode and the tissue in a body cavity.

2. The method according to claim 1, wherein the electrode comprises a working electrode and a reference electrode, and wherein the acquiring an impedance between the electrodes at different frequencies comprises: acquiring an impedance between the working electrodes, between the reference electrodes, and between the working electrode and the reference electrode at different frequencies.

3. The method according to claim 2, wherein the working electrode comprises an ablation electrode.

4. The method according to claim 2, wherein the determining a contact status between the electrode and the tissue in a body cavity comprises: determining a contact status between the working electrode and the tissue in the body cavity.

5. The method according to claim 1, wherein the body cavity invasion device comprises a catheter, and the electrode is provided at a distal end of the catheter.

6. The method according to claim 5, wherein the catheter comprises at least one of a basket-shaped catheter, a balloon-shaped catheter, a ring-shaped catheter, a petal-shaped catheter, a grid-shaped catheter, and a line-shaped catheter.

7. The method according to claim 1, wherein the different frequencies are selected according to the degree of difference how impedances of different tissues are in response to the frequencies.

8. The method according to claim 1, wherein the different frequencies range from 500 Hz to 100 kHz.

9. The method according to claim 1, wherein the different frequencies are two different frequencies.

10. The method according to claim 9, wherein the electrode comprises a working electrode and a reference electrode,

wherein the determining an impedance frequency response coefficient between the electrodes comprises: calculating an impedance frequency response coefficient between the working electrodes and an impedance frequency response coefficient between the reference electrodes according to equations (1) and (2), respectively: the impedance frequency response coefficient $\text{Coef}_{ij}$ between the working electrodes i and j is:

$$\text{Coef}_{ij} = a_{ij} \frac{\text{Impe}_{ij}^{\text{Fr1}}}{\text{Impe}_{ij}^{\text{Fr2}}} + b_{ij} \frac{\text{Real}_{ij}^{\text{Fr1}}}{\text{Real}_{ij}^{\text{Fr2}}} + c_{ij} \frac{\text{Imag}_{ij}^{\text{Fr1}}}{\text{Imag}_{ij}^{\text{Fr2}}} \quad ………………\quad (1)$$

the impedance frequency response coefficient $\text{Coef}_{mn}$ between the reference electrodes m and n is:

$$\text{Coef}_{mn} = a_{mn} \frac{\text{Impe}_{mn}^{\text{Fr1}}}{\text{Impe}_{mn}^{\text{Fr2}}} + b_{mn} \frac{\text{Real}_{mn}^{\text{Fr1}}}{\text{Real}_{mn}^{\text{Fr2}}} + c_{mn} \frac{\text{Imag}_{mn}^{\text{Fr1}}}{\text{Imag}_{mn}^{\text{Fr2}}} \quad ………………\quad (2)$$

wherein the determining a contact status between the electrode and the tissue in the body cavity comprises: calculating a contact index CI of the working electrodes i and j with the tissue according to equation (3):

$$CI = \frac{\left(\text{Coef}_{ij} - \text{Base}_{ij}\right) \big/ \text{Dis}_{ij}}{\left(\text{Coef}_{mn} - \text{Base}_{mn}\right) \big/ \text{Dis}_{mn}} \quad ………………………………\quad (3)$$

in the above equations (1), (2) and (3), $\text{Impe}_{ij}^{\text{Fr1}}$ denotes an impedance measured between the working electrodes i and j at a first frequency Fr1, $\text{Impe}_{ij}^{Fr2}$ denotes an impedance measured between the working electrodes i and j at a second frequency Fr2, $\text{Real}_{ij}^{Fr1}$ denotes a real part of a complex impedance measured between the working electrodes i and j at the first frequency Fr1, $\text{Real}_{ij}^{Fr2}$ denotes a real part of a complex impedance measured between the working electrodes i and j at the second frequency Fr2, $\text{Imag}_{ij}^{Fr1}$ denotes an imaginary part of the complex impedance measured between the working electrodes i and j at the first frequency Fr1, $\text{Imag}_{ij}^{Fr2}$ denotes an imaginary part of the complex impedance measured between the working electrodes i and j at the second frequency Fr2, $\text{Impe}_{mn}^{Fr1}$ denotes an impedance measured between the reference electrodes m and n at the first frequency Fr1, $\text{Impe}_{mn}^{Fr2}$ denotes an impedance measured between the reference electrodes m and n at the second frequency Fr2, $\text{Real}_{mn}^{Fr1}$ denotes a real part of a complex impedance measured between the reference electrodes m and n at the first frequency Fr1, $\text{Real}_{mn}^{Fr2}$ denotes a real part of a complex impedance measured between the reference electrodes m and n at the second frequency Fr2, $\text{Imag}_{mn}^{Fr1}$ denotes an imaginary part of the complex impedance measured between the reference electrodes m and n at the first frequency Fr1, $\text{Imag}_{mn}^{Fr2}$ denotes an imaginary part of the complex impedance measured between the reference electrodes m and n at the second frequency Fr2, $a_{ij}$, $b_{ij}$, $c_{ij}$, $a_{mn}$, $b_{mn}$, $c_{mn}$ are weight coefficients, $\text{Base}_{ij}$ is a base of a frequency response between the working electrodes i and j, $\text{Base}_{mn}$ is a base of a frequency response between the reference electrodes m and n, $\text{Dis}_{ij}$ is a distance between the working electrodes i and j, and $\text{Dis}_{mn}$ is a distance between the reference electrodes m and n.

11. The method according to claim 10, wherein the parameter $\text{Base}_{ij}$ and the parameter $\text{Base}_{mn}$ have correlation with the parameter $\text{Dis}_{ij}$ and the parameter $\text{Dis}_{mn}$.

12. The method according to claim 11, wherein the parameter $\text{Base}_{ij}$ and the parameter $\text{Base}_{mn}$ are obtained by performing high-order regression modeling analysis with the parameter $\text{Dis}_{ij}$, the parameter $\text{Dis}_{mn}$, a parameter for electrode width, and a parameter for electrode diameter.

13. The method according to claim 10, wherein magnitudes of the weight coefficients $a_{ij}$, $b_{ij}$, $c_{ij}$, $a_{mn}$, $b_{mn}$, $c_{mn}$ reflect weights of an impedance characteristic, a capacitive reactance characteristic and a comprehensive resistance-capacitance characteristic of the tissue or blood between the electrodes in an application environment, and $a_{ij} + b_{ij} + c_{ij} = 1$, $a_{mn} + b_{mn} + c_{mn} = 1$.

14. The method according to claim 1, further comprising: displaying the contact status between the electrode and the tissue in the body cavity with a color difference.

15. A system for identifying contact between an electrode and tissue, comprising:

an acquiring unit configured to acquire an impedance between the electrodes on a body cavity invasion device at different frequencies;
an impedance frequency response coefficient determining unit configured to determine, according to the acquired impedance between the electrodes, an impedance frequency response coefficient between the electrodes; and
a contact status determining unit configured to determine, according to the impedance frequency response coefficient, a contact status between the electrode and the tissue in a body cavity.

16. The system according to claim 15, wherein the electrode comprises a working electrode and a reference electrode, and
wherein the acquiring unit is configured to acquire an impedance between the working electrodes, between the reference electrodes, and between the working electrode and the reference electrode at different frequencies.

17. The system according to claim 16, wherein the working electrode comprises an ablation electrode.

18. The system according to claim 16, wherein the contact status determining unit is configured to determine a contact status between the working electrode and the tissue in the body cavity.

19. The system according to claim 15, wherein the body cavity invasion device comprises a catheter, and the electrode is provided at a distal end of the catheter.

20. The system according to claim 19, wherein the catheter comprises at least one of a basket-shaped catheter, a balloon-shaped catheter, a ring-shaped catheter, a petal-shaped catheter, a grid-shaped catheter, and a line-shaped catheter.

21. The system according to claim 15, wherein the different frequencies are selected according to the degree of difference how impedances of different tissues are in response to the frequencies.

22. The system according to claim 15, wherein the different frequencies range from 500 Hz to 100 kHz.

23. The system according to claim 15, wherein the different frequencies are two different frequencies.

24. The system according to claim 23, wherein the electrode comprises a working electrode and a reference electrode, and

wherein the impedance frequency response coefficient determining unit is configured to: calculate an impedance frequency response coefficient between the working electrodes and an impedance frequency response coefficient between the reference electrodes according to equations (1) and (2), respectively:
the impedance frequency response coefficient $Coef_{ij}$ between the working electrodes i and j is:

$$Coef_{ij} = a_{ij}\frac{Impe_{ij}^{Fr1}}{Impe_{ij}^{Fr2}} + b_{ij}\frac{Real_{ij}^{Fr1}}{Real_{ij}^{Fr2}} + c_{ij}\frac{Imag_{ij}^{Fr1}}{Imag_{ij}^{Fr2}} \quad …………… \quad （1）$$

the impedance frequency response coefficient $Coef_{mn}$ between the reference electrodes m and n is:

$$Coef_{mn} = a_{mn}\frac{Impe_{mn}^{Fr1}}{Impe_{mn}^{Fr2}} + b_{mn}\frac{Real_{mn}^{Fr1}}{Real_{mn}^{Fr2}} + c_{mn}\frac{Imag_{mn}^{Fr1}}{Imag_{mn}^{Fr2}} \quad ………… \quad （2）$$

wherein the contact status determining unit is configured to: calculate a contact index CI of the working electrodes i and j with the tissue according to equation (3):

$$CI = \frac{\left(Coef_{ij} - Base_{ij}\right)\big/Dis_{ij}}{\left(Coef_{mn} - Base_{mn}\right)\big/Dis_{mn}} \quad ……………………… \quad （3）$$

in the above equations (1), (2) and (3), $Impe_{ij}^{Fr1}$ denotes an impedance measured between the working electrodes i and j at a first frequency Fr1, $Impe_{ij}^{Fr2}$ denotes an impedance measured between the working electrodes i and j at a second frequency Fr2, $Real_{ij}^{Fr1}$ denotes a real part of a complex impedance measured between the working electrodes i and j at the first frequency Fr1, $Real_{ij}^{Fr2}$ denotes a real part of a complex impedance measured between the working electrodes i and j at the second frequency Fr2, $Imag_{ij}^{Fr1}$ denotes an imaginary part of the complex impedance measured between the working electrodes i and j at the first

frequency Fr1, $\mathrm{Imag}_{ij}^{Fr2}$ denotes an imaginary part of the complex impedance measured between the working electrodes i and j at the second frequency Fr2, $\overline{\mathrm{Impe}}_{mn}^{Fr1}$ denotes an impedance measured between the reference electrodes m and n at the first frequency Fr1, $\overline{\mathrm{Impe}}_{mn}^{Fr2}$ denotes an impedance measured between the reference electrodes m and n at the second frequency Fr2, $\mathrm{Real}_{mn}^{Fr1}$ denotes a real part of a complex impedance measured between the reference electrodes m and n at the first frequency Fr1, $\mathrm{Real}_{mn}^{Fr2}$ denotes a real part of a complex impedance measured between the reference electrodes m and n at the second frequency Fr2, $\overline{\mathrm{Imag}}_{mn}^{Fr1}$ denotes an imaginary part of the complex impedance measured between the reference electrodes m and n at the first frequency Fr1, $\overline{\mathrm{Imag}}_{mn}^{Fr2}$ denotes an imaginary part of the complex impedance measured between the reference electrodes m and n at the second frequency Fr2, $a_{ij}$, $b_{ij}$, $c_{ij}$, $a_{mn}$, $b_{mn}$, $c_{mn}$ are weight coefficients, $Base_{ij}$ is a base of a frequency response between the working electrodes i and j, $Base_{mn}$ is a base of a frequency response between the reference electrodes m and n, $Dis_{ij}$ is a distance between the working electrodes i and j, and $Dis_{mn}$ is a distance between the reference electrodes m and n.

25. The system according to claim 24, wherein the parameter $Base_{ij}$ and the parameter $Base_{mn}$ have correlation with the parameter $Dis_{ij}$ and the parameter $Dis_{mn}$.

26. The system according to claim 25, wherein the parameter $Base_{ij}$ and the parameter $Base_{mn}$ are obtained by performing high-order regression modeling analysis with the parameter $Dis_{ij}$, the parameter $Dis_{mn}$, a parameter for electrode width and a parameter for electrode diameter.

27. The system according to claim 24, wherein magnitudes of the weight coefficients $a_{ij}$, $b_{ij}$, $c_{ij}$, $a_{mn}$, $b_{mn}$, $c_{mn}$ reflects weights of an impedance characteristic, a capacitive reactance characteristic and a comprehensive resistance-capacitance characteristic of the tissue or blood between the electrodes in an application environment, and $a_{ij} + b_{ij} + c_{ij} = 1$, $a_{mn} + b_{mn} + C_{mn} = 1$.

28. The system according to claim 15, further comprising a display unit configured to display the contact status between the electrode and the tissue in the body cavity with a color difference.

29. A computer readable medium having stored thereon instructions executable by a processor, the instructions, when executed by the processor, causing the processor to perform the method for identifying contact between an electrode and tissue according to claim 1.

FIG. 1

FIG. 2

FIG. 3A

RSPV

LSPV

RIPV

LIPV

310

308

309

304

305

313

311

312

FIG.3B

FIG. 4A

FIG. 4B

500

Acquiring an impedance between electrodes at
a first frequency and a second frequency,
respectively — 502

Calculating an impedance frequency response
coefficient between working electrodes — 504

Calculating an impedance frequency response
coefficient between reference electrodes — 506

Calculating a contact (sticking) index CI — 508

Outputting and displaying an identification
result of contact between the electrode and
the tissue — 510

FIG.5

FIG. 6

<u>**700**</u>

S710

Acquiring an impedance between the electrodes on a body cavity invasion device at different frequencies

S720

Determining, according to the acquired impedance between the electrodes, an impedance frequency response coefficient between the electrodes

S730

Determining, according to the impedance frequency response coefficient, a contact status between the electrode and the tissue in a body cavity

FIG. 7

<u>**800**</u>

810

820

830

Acquiring Unit → Impedance Frequency Response Coefficient Determining Unit → Contact Status Determining Unit

FIG. 8

## INTERNATIONAL SEARCH REPORT

| International application No. |
| --- |
| **PCT/CN2023/094825** |

**A. CLASSIFICATION OF SUBJECT MATTER**

A61B18/14(2006.01)i

According to International Patent Classification (IPC) or to both national classification and IPC

**B. FIELDS SEARCHED**

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT; CNKI; ENTXTC; ENTXT; OETXT; VEN: 电极, 组织, 接触, 触摸, 接近, 触碰, 靠近, 贴近, 贴合, 识别, 检测, 监测, 确定, 频率, 阻抗, 复阻抗, 响应, 系数, 比率, 比值, electrode, touch, close, identify, detect, frequency, impedance, coefficient, ratio

**C. DOCUMENTS CONSIDERED TO BE RELEVANT**

| Category* | Citation of document, with indication, where appropriate, of the relevant passages | Relevant to claim No. |
| --- | --- | --- |
| X | CN 105615993 A (BIOSENSE WEBSTER (ISRAEL) LTD.) 01 June 2016 (2016-06-01) description, paragraphs [0041]-[0103], and figures 1-16 | 1-9, 14-23, 28, 29 |
| X | CN 107148249 A (ADVANCED CARDIAC THERAPEUTICS, INC.) 08 September 2017 (2017-09-08) description, paragraphs [0235]-[0496], and figures 1-34C | 1-9, 14-23, 28, 29 |
| X | CN 111096749 A (BIOSENSE WEBSTER (ISRAEL) LTD.) 05 May 2020 (2020-05-05) description, paragraphs [0021]-[0051], and figures 1-3 | 1-9, 14-23, 28, 29 |
| A | CN 107635463 A (NAVIX INTERNATIONAL LTD.) 26 January 2018 (2018-01-26) entire document | 1-29 |
| A | CN 110300559 A (MEDTRONIC INC.) 01 October 2019 (2019-10-01) entire document | 1-29 |
| A | CN 110537970 A (BIOSENSE WEBSTER (ISRAEL) LTD.) 06 December 2019 (2019-12-06) entire document | 1-29 |

☑ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **15 August 2023** | **28 August 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/094825**

## C.    DOCUMENTS CONSIDERED TO BE RELEVANT

| Category* | Citation of document, with indication, where appropriate, of the relevant passages | Relevant to claim No. |
|---|---|---|
| A | US 2009275827 A1 (AIKEN ROBERT D et al.) 05 November 2009 (2009-11-05) entire document | 1-29 |

Form PCT/ISA/210 (second sheet) (July 2022)

**INTERNATIONAL SEARCH REPORT**
Information on patent family members

International application No.

**PCT/CN2023/094825**

| Patent document cited in search report | | | Publication date (day/month/year) | Patent family member(s) | | | Publication date (day/month/year) |
|---|---|---|---|---|---|---|---|
| CN | 105615993 | A | 01 June 2016 | None | | | |
| CN | 107148249 | A | 08 September 2017 | None | | | |
| CN | 111096749 | A | 05 May 2020 | None | | | |
| CN | 107635463 | A | 26 January 2018 | None | | | |
| CN | 110300559 | A | 01 October 2019 | None | | | |
| CN | 110537970 | A | 06 December 2019 | None | | | |
| US | 2009275827 | A1 | 05 November 2009 | US | 10362959 | B2 | 30 July 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210541390